# EUROPEAN PATENT SPECIFICATION

(11) **EP 3 318 303 B1**
(45) Date of publication and mention of the grant of the patent: **11.09.2019**
(21) Application number: 16820682.9
(22) Date of filing: 15.04.2016
(51) Int. Cl.: A61M 16/20

(54) **INTEGRAL TYPE OXYGEN MIXING CHANNEL FOR RESPIRATOR OXYGEN MIXING VALVE**
INTEGRIERTER SAUERSTOFFMISCHKANAL FÜR ATEMSCHUTZSAUERSTOFF-MISCHVENTIL
CANAL MONOBLOC DE MÉLANGE D'OXYGÈNE POUR SOUPAPE DE MÉLANGE D'OXYGÈNE DE RESPIRATEUR

(30) Priority: 06.07.2015 CN 201520479627 U
(43) Date of publication of application: 09.05.2018
(73) Proprietor: Beijing Aeonmed Co., Ltd., Beijing 100070 (CN)
(72) Inventor: ZHANG, Xinjian, Beijing 100070 (CN); MA, Yingdan, Beijing 100070 (CN)
(74) Representative: Hanna Moore + Curley
(86) International application number: PCT/CN2016/079340
(87) International publication number: WO 2017/005031

(56) References cited:
- CN-A- 101 025 235
- CN-A- 104 208 794
- CN-A- 104 208 794
- CN-Y- 200 991 483
- CN-Y- 200 991 483
- FR-A1- 2 887 950
- JP-A- H07 158 766
- US-A- 4 917 137

## Description

### TECHNICAL FIELD

The present invention relates to the field of breathing machine equipment, specifically to an integral oxygen mixing channel of an oxygen mixing valve of a breathing machine.

### BACKGROUND OF THE INVENTION

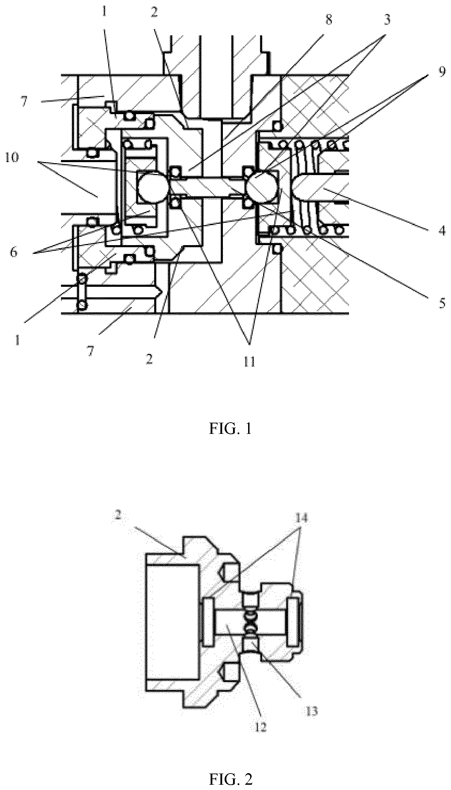

An oxygen mixing valve is a key component of a breathing machine, with a function of mixing air and oxygen at a set ratio and then providing them to a patient, which requires a higher accuracy on its adjustment of a concentration of oxygen in the mixed gas. Although a feedback adjustment by a software can guarantee a precision of the adjustment, the software cannot adjust the concentration of oxygen to a set value in the case of an oversize deviation. The adjustment of the concentration of oxygen within a controllable range requires to be ensured by a structure of the oxygen mixing valve. Currently, the structures of the oxygen mixing valves are mostly as shown in Fig. 1.

An adjustment rod 4 pushes a steel ball guide seat 6 on its left to control an opening of the steel ball guide seat 6. An ejector rod 5 pushes another steel ball guide seat 6 as the left steel ball guide seat 6 correspondingly moves. The degree of opening of the steel ball guide seats at both sides decides the ratio of both gases of air entering from an air channel 9 and oxygen entering from an oxygen channel 10 into an oxygen mixing chamber 8. A distance for effective adjustment of a gas mixing ratio on this structure is the distance between two seal rings 3.

The above structure has a certain defect, since installation grooves of the seal rings are on an adjustable piston 2 and a valve body 7, respectively, and an adjustable piston sleeve 1 is connected to the adjustable piston 2 via an internal thread and to the valve body 7 via an external thread. Such structure after assembled may lead to error accumulation (machining error and assembly error), unable to ensure the effective adjustment of the distance, and thus causing trouble in debugging of the oxygen mixing valve on a production line, which requires to repeatedly dismount and install the assembly of the valve body to adjust the distance of the adjustable piston sleeve screwed in or out of the adjustable piston, and thereafter also requires to adjust the in and out of the adjustment rod, with a low adjustment efficiency. It is often difficult to determine which end should be specifically adjusted, and the appropriate degree for adjustment during the adjustment at both ends. CN104208794 describes a oxygen mixing valve and a breathing machine.

### SUMMARY OF THE INVENTION

The present invention directs at the defect of the above structure, in which the structure of the adjustable piston is modified, the oxygen mixing channel is modified into an integral structure, the seal ring grooves are designed onto a same part, and the distance between the two seal rings is ensured with a machining method, such that the adjustable piston may be assembled at one time without the need of adjustment, and the assembly of the valve body does not need to be dismounted and installed in debugging with the only need of in-and-out adjustment for the adjustment rod to complete the debugging. Therefore, the difficulties in debugging can be greatly reduced.

An integral oxygen mixing channel of an oxygen mixing valve of a breathing machine of the present invention comprises an adjustable piston sleeve 1, an adjustable piston 2 and a valve body 7, the adjustable piston sleeve 1 being connected to one end of the adjustable piston 2 via an internal thread, and then being connected to the valve body 7 via an external thread, wherein the other end of the adjustable piston 2 penetrates through the valve body 7;
both ends of the adjustable piston 2 are coaxially provided with a pair of seal ring grooves 14, respectively, the seal ring grooves 14 are placed with seal rings 3 therein, and an area of the adjustable piston 2 between the two seal rings 3 is designed into a hollow cylindrical shape to form an oxygen mixing channel 12; and
a circumference of the oxygen mixing channel 12 is provided with several exhaust holes 13.

According to the integral oxygen mixing channel of the present invention, several exhaust holes 13 are preferably provided evenly along a middle segment of the circumference of the oxygen mixing channel 12. Further most preferably, six exhaust holes 13 are provided evenly along the middle segment of the circumference of the oxygen mixing channel 12.

The integral oxygen mixing channel according to the present invention further comprises a pair of steel balls 11 cooperatively disposed in one-to-one opposition with the two seal rings 3, respectively, and there are adjustable air inlet gaps between the steel balls 11 and the seal rings 3. A total area of the several exhaust holes is greater than a sum of the air inlet gaps between the steel balls 11 and the seal rings 3.

Preferably, a supportive fixation is provided depending on a compression of the seal rings, between the adjustable piston 2 and the valve body 7.

The integral oxygen mixing channel further comprises an ejector rod 5 located within the oxygen mixing channel 12.

With the invention, an oxygen channel, an air channel and the two seal ring grooves are all designed on the adjustable piston, and the distance between the two seal rings and a coaxiality thereof are ensured through a dimensional tolerance and a geometric tolerance. The ejector rod is installed into the oxygen mixing channel of the adjustable piston, and a sufficient air inlet gap from the ejector rod is left by calculating and designing an inner diameter of the oxygen mixing channel. The exhaust holes are opened along the circumference of the oxygen mixing channel, with the total area of the exhaust holes greater than the maximum air inlet gaps between the steel balls and the seal rings. This structure can ensure a controllability of adjustment of the concentration of the mixed oxygen in terms of a mechanism, greatly simplifying a debugging process of a production line. The adjustable piston is assembled at one time without the need of adjustment, and the adjustment rod is only required to be adjusted, i.e., the debugging may be accomplished by the adjustment at one end, greatly reducing the difficulty in debugging and improving the production efficiency. Furthermore, in addition to the mixing of air and oxygen in a breathing machine, the present invention can also be used for other devices that need to mix any two gases at a ratio.

### BRIEF DESCRIPTION OF THE DRAWINGS

Fig. 1 is a cross-sectional schematic diagram of an existing oxygen mixing channel of an oxygen mixing valve of a breathing machine.
Fig. 2 is a cross-sectional schematic diagram of an adjustable piston and an oxygen mixing channel of the present invention.
Fig. 3 is a cross-sectional schematic diagram of an oxygen mixing channel of an oxygen mixing valve of a breathing machine of the present invention.

**Reference Numbers**

| | | | |
|---|---|---|---|
| 1. adjustable piston sleeve | 2. adjustable piston | 3. seal ring | 4. adjustment rod |
| 5. ejector rod | 6. steel ball guide seat | 7. valve body | 8. oxygen mixing chamber |
| 9. air channel | 10. oxygen channel | 11. steel ball | 12. oxygen mixing channel |
| 13. exhaust hole | 14. seal ring groove | | |

### DETAILED DESCRIPTION OF THE INVENTION

The present invention is now further described in detail in conjunction with the drawings. The drawings are all simplified schematic diagrams, illustrating the basic structure of the present invention only in a schematic manner, which therefore only show the constitutions related to the present invention.

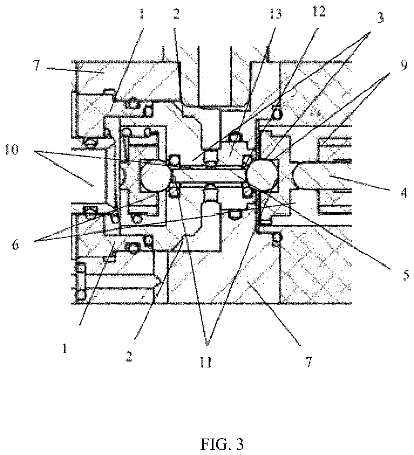

As shown in Fig. 3, an integral oxygen mixing channel of an oxygen mixing valve of a breathing machine of the present invention comprises an adjustable piston sleeve 1, an adjustable piston 2 and a valve body 7, the adjustable piston sleeve 1 being connected to one end of the adjustable piston 2 via an internal thread, and then being connected to the valve body 7 via an external thread, and wherein the other end of the adjustable piston 2 penetrates through the valve body 7. As shown in Fig. 2, both ends of the adjustable piston 2 are coaxially provided with a pair of seal ring grooves 14, respectively, the seal ring grooves 14 are placed with seal rings 3 therein, and an area of the adjustable piston 2 between the two seal rings 3 is designed into a hollow cylindrical shape to form an oxygen mixing channel 12; and a circumference of the oxygen mixing channel 12 is provided with several exhaust holes 13.

Preferably, several exhaust holes 13 are provided evenly along a middle segment of the circumference of the oxygen mixing channel 12. Most preferably, six exhaust holes 13 are provided evenly along the middle segment of the circumference of the oxygen mixing channel 12.

The integral oxygen mixing channel according to the present invention further comprises a pair of steel balls 11 cooperatively disposed in one-to-one opposition with the two seal rings 3, respectively, and there are adjustable air inlet gaps between the steel balls 11 and the seal rings 3. A total area of the several exhaust holes is greater than a sum of the air inlet gaps between the steel balls 11 and the seal rings 3.

Preferably, a supportive fixation is provided while a seal is ensured depending on a compression of the seal rings, between the adjustable piston 2 and the valve body 7.

The integral oxygen mixing channel further comprises an ejector rod 5 located within the oxygen mixing channel 12.

The specific operating principle of the oxygen mixing channel of the oxygen mixing valve of the breathing machine of the present invention is shown as follows:

The modified assembly structure of the present invention is shown in Fig. 3, an oxygen channel, an air channel, and the two seal ring grooves are all designed on the adjustable piston, the oxygen mixing channel is modified into an integral structure, the seal ring grooves are designed onto a same part, and the distance between the two seal rings is ensured with a machining method, such that the adjustable piston may be assembled at one time without the need of adjustment, and only adjustment to the adjustment rod is required in debugging to complete the debugging. Therefore, the difficulties in debugging can be greatly reduced. The distance between the two seal rings and a coaxiality thereof are ensured through a dimensional tolerance. The ejector rod is installed into the oxygen mixing channel of the adjustable piston, and a sufficient air inlet gap from the ejector rod is left by calculating and designing an inner diameter of the oxygen mixing channel. The exhaust holes are opened along the circumference of the oxygen mixing channel, with the total area of the exhaust holes greater than the maximum air inlet gaps between the steel balls and the seal rings. This structure can ensure a controllability of adjustment of the concentration of the mixed oxygen in terms of a mechanism, greatly simplifying a debugging process of a production line. The adjustable piston is assembled at one time without the need of adjustment, and the adjustment rod is only required to be adjusted, i.e., the debugging may be accomplished by the adjustment at one end, greatly reducing the difficulty in debugging and improving the production efficiency.

Of course, the present invention can also have a variety of embodiments. Those skilled in the art can make various respective changes and variations according to the disclosure of the present invention without departing from the essence of the present invention, and these respective changes and variations shall all belong to the protection scope of the appended claims of the present invention.

## Claims

1. An integral type oxygen mixing channel of an oxygen mixing valve of a breathing machine, comprising an adjustable piston sleeve (1), an adjustable piston (2) and a valve body (7), the adjustable piston sleeve (1) being connected to one end of the adjustable piston (2) via an internal thread, and then being connected to the valve body (7) via an external thread,
wherein the other end of the adjustable piston (2) penetrates through the valve body (7);
both ends of the adjustable piston (2) are coaxially provided with a pair of seal ring grooves (14), respectively, the seal ring grooves (14) are placed with seal rings (3) therein, and an area of the adjustable piston (2) between the two seal rings (3) is designed into a hollow cylindrical shape to form an oxygen mixing channel (12); and
a circumference of the oxygen mixing channel (12) is provided with several exhaust holes (13).

2. The integral type oxygen mixing channel according to claim 1, wherein several exhaust holes (13) are provided evenly along a middle segment of the circumference of the oxygen mixing channel (12).

3. The integral type oxygen mixing channel according to claim 2, wherein six exhaust holes (13) are provided evenly along the middle segment of the circumference of the oxygen mixing channel (12).

4. The integral type oxygen mixing channel according to claims 1-3, wherein the integral oxygen mixing channel further comprises a pair of steel balls (11) cooperatively disposed in one-to-one opposition with the two seal rings (3), respectively, and there are adjustable air inlet gaps between the steel balls (11) and the seal rings (3).

5. The integral type oxygen mixing channel according to claim 4, wherein a total area of the several exhaust holes is greater than a sum of the air inlet gaps between the steel balls (11) and the seal rings (3).

6. The integral type oxygen mixing channel according to claim 1, wherein a supportive fixation is provided depending on a compression of the seal rings, between the adjustable piston (2) and the valve body (7).

7. The integral type oxygen mixing channel according to claim 1, wherein the integral oxygen mixing channel further comprises an ejector rod (5) located within the oxygen mixing channel (12).

## Patentansprüche

1. Integraler Sauerstoffmischkanal eines Sauerstoffmischventils eines Atemgeräts, umfassend eine justierbare Kolbenmanschette (1), einen justierbaren Kolben (2) und einen Ventilkörper (7), wobei die justierbare Kolbenmanschette (1) über ein Innengewinde mit einem Ende des justierbaren Kolbens (2) verbunden ist und dann über ein Außengewinde mit dem Ventilkörper (7) verbunden ist,
wobei das andere Ende des justierbaren Kolbens (2) den Ventilkörper (7) durchdringt;
wobei beide Enden des justierbaren Kolbens (2) jeweils koaxial mit einem Paar von Dichtungsringnuten (14) versehen sind, wobei Dichtungsringe (3) in den Dichtungsringnuten (14) platziert sind und ein Bereich des justierbaren Kolbens (2) zwischen den zwei Dichtungsringen (3) in eine Hohlzylinderform konstruiert ist, um einen Sauerstoffmischkanal (12) zu bilden; und
ein Umfang des Sauerstoffmischkanals (12) mit mehreren Abgaslöchern (13) versehen ist.

2. Integraler Sauerstoffmischkanal nach Anspruch 1, wobei mehrere Abgaslöcher (13) gleichmäßig entlang eines Mittelsegments des Umfangs des Sauerstoffmischkanals (12) vorgesehen sind.

3. Integraler Sauerstoffmischkanal nach Anspruch 2, wobei sechs Abgaslöcher (13) gleichmäßig entlang des Mittelsegments des Umfangs des Sauerstoffmischkanals (12) vorgesehen sind.

4. Integraler Sauerstoffmischkanal nach den Ansprüchen 1-3, wobei der integrale Sauerstoffmischkanal ferner ein Paar von Stahlkugeln (11) umfasst, die jeweils zusammenwirkend eins-zu-eins den zwei Dichtungsringen (3) gegenüberliegend angeordnet sind, und wobei es zwischen den Stahlkugeln (11) und den Dichtungsringen (3) justierbare Lufteinlassspalten gibt.

5. Integraler Sauerstoffmischkanal nach Anspruch 4, wobei eine Gesamtfläche der mehreren Abgaslöcher größer als eine Summe der Lufteinlassspalten zwischen den Stahlkugeln (11) und den Dichtungsringen (3) ist.

6. Integraler Sauerstoffmischkanal nach Anspruch 1, wobei eine stützende Fixierung abhängig von einem Zusammendrücken der Dichtungsringe bereitgestellt wird, zwischen dem justierbaren Kolben (2) und dem Ventilkörper (7).

7. Integraler Sauerstoffmischkanal nach Anspruch 1, wobei der integrale Sauerstoffmischkanal ferner eine Auswerferstange (5) umfast, die innerhalb des Sauerstoffmischkanals (12) angeordnet ist.

## Revendications

1. Canal monobloc de mélange d'oxygène d'une soupape de mélange d'oxygène d'une machine respiratoire, comprenant un manchon de piston ajustable (1), un piston ajustable (2) et un corps de soupape (7), le manchon de piston ajustable (1) étant relié à une extrémité du piston ajustable (2) par l'intermédiaire d'un filetage interne, puis étant relié au corps de soupape (7) par l'intermédiaire d'un filetage externe,
l'autre extrémité du piston ajustable (2) pénétrant à travers le corps de soupape (7) ;
les deux extrémités du piston ajustable (2) comportant, coaxialement, une paire de rainures de bague d'étanchéité (14), respectivement, les rainures de bague d'étanchéité (14) recevant des bagues d'étanchéité (3) dans celles-ci, et une zone du piston ajustable (2) entre les deux bagues d'étanchéité (3) étant agencée en une forme cylindrique creuse pour former un canal de mélange d'oxygène (12) ; et
une circonférence du canal de mélange d'oxygène (12) comportant plusieurs trous d'échappement (13).

2. Canal monobloc de mélange d'oxygène selon la revendication 1, dans lequel plusieurs trous d'échappement (13) sont disposés de manière régulière le long d'un segment central de la circonférence du canal de mélange d'oxygène (12).

3. Canal monobloc de mélange d'oxygène selon la revendication 2, dans lequel six trous d'échappement (13) sont disposés de manière régulière le long du segment central de la circonférence du canal de mélange d'oxygène (12) .

4. Canal monobloc de mélange d'oxygène selon les revendications 1 à 3, le canal monobloc de mélange d'oxygène comprenant en outre une paire de billes d'acier (11) disposées de manière coopérative en opposition une-à-une avec les deux bagues d'étanchéité (3), respectivement, et il y a des intervalles d'entrée d'air ajustables entre les billes d'acier (11) et les bagues d'étanchéité (3).

5. Canal monobloc de mélange d'oxygène selon la revendication 4, dans lequel une aire totale des plusieurs trous d'échappement est supérieure à une somme des intervalles d'entrée d'air entre les billes d'acier (11) et les bagues d'étanchéité (3).

6. Canal monobloc de mélange d'oxygène selon la revendication 1, dans lequel une fixation de support est prévue, fonction d'une compression des bagues d'étanchéité, entre le piston ajustable (2) et le corps de soupape (7).

7. Canal monobloc de mélange d'oxygène selon la revendication 1, le canal monobloc de mélange d'oxygène comprenant en outre une tige d'éjecteur (5) située à l'intérieur du canal de mélange d'oxygène (12).
